# EUROPEAN PATENT APPLICATION

(11) **EP 1 769 768 A1**
(43) Date of publication of application: **04.04.2007**
(21) Application number: 05256031.5
(22) Date of filing: 28.09.2005
(51) Int. Cl.: A61B 19/00

(54) **Surgical instrument calibration**

(71) Applicant: DePuy Orthopädie GmbH, 85551 Heimstetten (DE); Kennedy, James, Berkley, MA 02779 (US); Livorsi, Carl, Lakeville, MA 02347 (US); Howell, Chris, Sandwich, MA 02563 (US)
(72) Inventor: The designation of the inventor has not yet been filed
(74) Representative: Alton, Andrew

(57) **Abstract**

A rotationally symmetric surgical instrument for use with a computer aided surgery system which is adapted so that it is easy to check its calibration and methods of checking the calibration of such instruments are described. The instrument includes a body having a working end which includes a working point which characterises the location in space of the working end. The instrument also has a location at which a marker can be attached to the instrument. Only a single formation is located toward the working end which provides a calibration reference point for engaging with a trackable instrument. The single formation has a fixed position relative to the working point. The position of the calibration reference point relative to the marker array can be determined so as to check whether the calibration of the instrument is still accurate.

## Description

The present invention relates to surgical instruments, and in particular to a method for checking the calibration of a surgical instrument for use with a computer aided surgery system and an instrument adapted for use in such a method.

Computer aided, or assisted, surgery (CAS) often involves the use of instruments, implants and other entities whose position is tracked in real time so as to provide navigated positioning of the entities to guide the surgeon carrying out the operation. The entities being tracked include a marker, also referred to as a fiducial, by which a tracking part of the CAS system can identify and track the real time position of the entity. Prior to use, the entity is usually calibrated so that the CAS system has stored data indicating the relative position of a working part of the entity, e.g. the tip of a pointer, and the position of a marker attached to the entity. Hence, the position of the working part of the entity can be determined from the tracked position of the marker.

Various methods can be used in order to calibrate an instrument during a surgical procedure. One method is to use a calibration matrix which has a trackable marker attached thereto. A rotationally symmetric marked instrument is located within the matrix and rotated about its longitudinal axis so that the direction of the axis of the instrument can be determined. Additionally the tip of the tool is held against a defined surface of the calibration matrix so as to determine the position of the tip of the tool relative to the marker on the tool. However, such a calibration matrix is complex and expensive to manufacture owing to the accuracy required. Also this approach takes significant time and it is preferred to carry out a surgical procedure as quickly as possible and without introducing delays.

An alternate approach for any type of instrument is to provide three points at defined positions on the instrument relative to a marker array. Then the instrument can be calibrated by placing the tip of a trackable pointer on each of the three points, determining the positions of the three points and then determining the position of the instrument relative to the marker array. However, this still requires a significant delay while the positions of the three points are captured.

Ideally there would be no requirement to check the calibration of calibrated instruments at all as it simply delays the surgical procedure.

However, pre-calibrated instruments are difficult to manufacture to the high levels of tolerance required. Further, although originally their calibration may be correct, the accuracy of calibration of the instruments can reduce as they are used owing to wear and tear or damage.

Hence, it would be advantageous to be able to quickly and easily check the calibration of a surgical instrument. It would also be beneficial to be able to correct the calibration of the instrument. It would further be beneficial if no specialized equipment were required in order to do so.

According to a first aspect of the present invention, there is provided a surgical instrument for use with a computer aided surgery system. The instrument has a working end and a working point which characterises the location in space of the working end. The instrument also has a location at which a marker can be or is attached to the instrument. The instrument includes only a single formation which provides a calibration reference point and is located toward the working end. The formation has a substantially fixed position relative to the working point and can be engaged by a trackable instrument so that the position of the calibration reference point can be determined.

As there is a fixed and known positional relationship between the calibration formation and the working point, by determining the position of the calibration formation, the calibration of the instrument can be checked.

Preferably the formation is adapted to engage with a trackable instrument in use so that the position of the calibration reference point can be determined from the tracked position of the instrument. The formation can be adapted to mate with a part of the trackable instrument. The formation can be a male or female formation. Preferably, the formation is a recessed formation for example a dimple. Preferably, the formation is shaped and/or sized so as to match a part of an instrument, eg a tip of a pointer, so as to allow accurate registration of the part of the instrument and the formation.

The calibration reference point can be coincidental with the working point.

The calibration reference point can be on a longitudinal axis of the instrument.

The calibration reference point can be off set from a longitudinal axis of the instrument.

The calibration reference point can be located at the working point or toward, near or adjacent to the working end and working point of the instrument. The calibration point can be located within half of the longitudinal length of the instrument from the working point, preferably within a third of the longitudinal length, more preferably within a quarter, fifth or sixth of the longitudinal length. Most preferably, the calibration reference point is positioned on the instrument so as to reduce or minimise the likelihood of use, damage or wear of the instrument changing the positional relationship between the calibration reference point and the working point.

According to a further aspect of the invention, there is provided a computer implemented method for checking the calibration of a surgical instrument being used with a computer aided surgery system. The method can comprise determining the position of a trackable marker attached to the instrument; determining the position of a single formation defining a calibration reference point of the instrument from the tracked position of a further instrument while engaging the formation; and using the position of the calibration reference point to determine whether the actual position of the working point relative to the position of the trackable marker corresponds to a calibration position of the working point relative to the position of the trackable marker.

If the actual position does not correspond to the calibration position, then new calibration data representing the actual position of the working point relative to the position of the trackable marker can be stored. The new calibration data can be used to determine the position of the working point subsequently.

The method can further comprise reading or accessing stored data representing the position of the calibration reference point relative to the working point. This allows the offset between the calibration reference point and working point to be determined.

According to a further aspect of the invention, there is provided a computer aided surgery system which can check the calibration of a surgical instrument being used with the computer aided surgery system. The computer aided surgery system can include a data processing device and a medium storing computer program code providing instructions which can cause the system to: determine the position of a trackable marker attached to the instrument; determine the position of the calibration reference point of the instrument from the tracked position of a further instrument which engages a formation defining the calibration reference point; and use the position of the calibration reference point to determine whether the actual position of the working point relative to the position of the trackable marker corresponds to a calibration position of the working point relative to the position of the trackable marker.

According to a further aspect of the invention, there is provided a method for checking the calibration of a rotationally symmetric instrument during a computer aided surgical procedure. The method can comprise engaging a further trackable instrument with only a single formation on the surgical instrument which defines a calibration reference point. The method can further comprise causing the position of the calibration reference point to be determined. Causing the position to be determined can include manipulating the trackable instrument, for example by pivoting about the formation, actuating a switch or otherwise entering an instruction or command to cause data to be captured or entered. The further trackable instrument can be a pointer bearing a marker.

The method can further comprise instructing a computer aided surgery system whether to re-calibrate the surgical instrument, for example by inputting a command or instruction.

According to further aspects of the invention, there are provided a computer program code executable by a data processing device to carry out the method aspects of the invention or the computer aided surgery system aspect of the invention. A computer readable medium bearing such computer program code is also provided,

An embodiment of the invention will now be described, by way of example only, and with reference to the accompanying drawings, in which:
Figure 1 shows a schematic block diagram of a CAS system according to the present invention;
Figure 2 shows a schematic diagram of a prior art instrument;
Figures 3 and 4 show schematic diagrams of an instrument according to the invention illustrating the method of the invention;
Figure 5 shows a flow chart illustrating a method of checking the calibration of an instrument according to the present invention;
Figures 6 and 7 show schematic diagrams of a further instrument according to the invention illustrating the method of the invention; and
Figure 8 shows a process flow chart illustrating a computer implemented method of checking the calibration of an instrument according to the invention;

The same or similar items in different Figures share common reference numerals unless indicated otherwise.

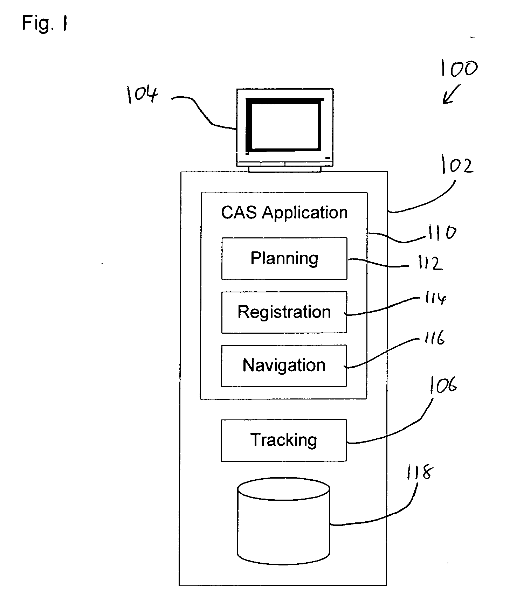

Figure 1 shows a schematic block diagram of a CAS system 100 according to the invention with which an instrument according to the invention can be used according to various methods also according to the invention. CAS system 100 includes a data processing apparatus 102 in the form of a general purpose computer including a display device 104. CAS system 100 also includes a tracking subsystem 106 for tracking the positions of various suitably marked entities, such as surgical instruments, implants, tools and any other devices commonly used in a surgical procedure. The tracking subsystem provides tracking data to the CAS system which identifies each entity being tracked and which specifies the position and orientation of any of the entities with a reference frame of the tracking system.

Various tracking technologies can be used such as wire based or wireless tracking technologies. For example acoustic based tracking systems can use ultrasound whereas electro-magnetic based tracking systems can use radio frequency, infra red or other portions of the electromagnetic spectrum. In one embodiment an infrared based tracking system is used comprising a pair of off-set infrared cameras capture images of marker arrays, also referred to as star arrays or simply stars, comprising trios of infrared reflective spheres. Other arrangements of markers can also be used.

The CAS system generally includes a computer aided surgery application 110 which generally defines a workflow which guides the surgeon through the various operations involved in using the CAS system to carry out a surgical procedure. The CAS application can include a number of functions. For example, a planning module 112 can be provided which the surgeon uses to help plan the surgical procedure. A registration module 114 can be provided which is used to register the position of the patient in the reference frame of the tracking system and/or an image or images with the position of the patient. A navigation module 116 can also be provided which helps to navigate the various entities being used by the surgeon, for example in an Image Guided Surgery (IGS) approach. The CAS application can interact with a database 118 which stores data relating to the entities used by the CAS system such as properties or attributes of the entities being tracked, such as calibration data, and also images of the entities being tracked so that 3D renderings of the entities can be displayed during navigation.

The invention allows the surgeon to check the calibration of a rotationally symmetric instrument to be used in the procedure. As used herein, calibration relates to the fact that the tracking system does not directly track the position of the working part of the instrument, e.g. the tip of a reamer or inserter. Rather the tracking system tracks the position of the marker and then determines the position of the working part or point of the instrument from calibration data which defines the positional relationship between the tracked marker and the working point.

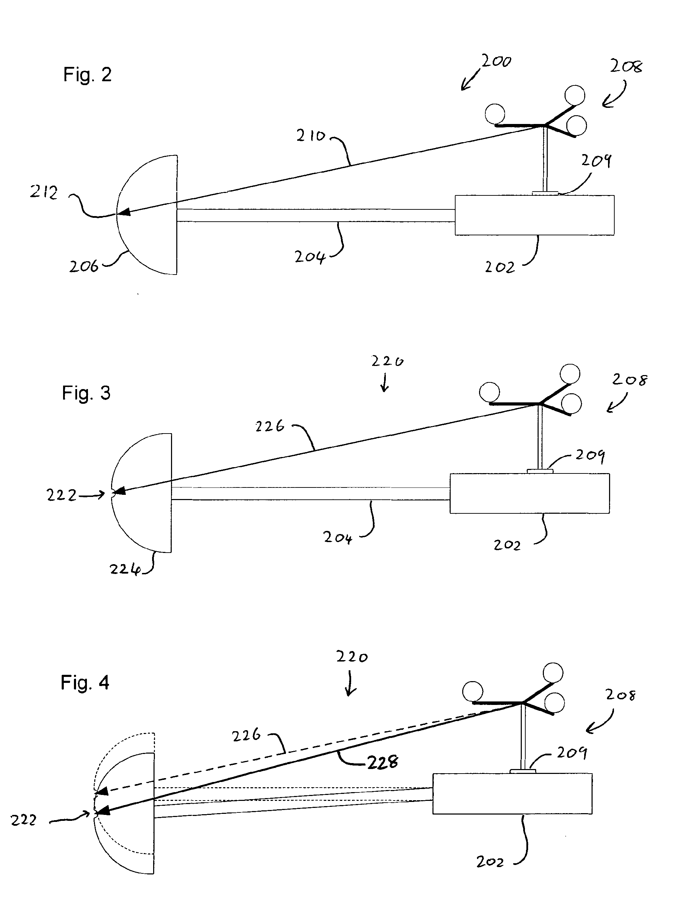

For example, Figure 2 shows a schematic illustration of a prior art rotationally symmetric instrument 200 in the form of an acetabular cup inserter. The instrument 200 includes a handle 202, a body 204 extending from the handle and an acetabular cup 206 mounted on the distal end of the instrument. A marker in the form of an array, or star array, 208 is attached to the handle by a connector 209 at a marker attachment point or position.

The marked instrument is pre-calibrated, as represented by vector 210 which represents the positional relationship between the centre of the array 208 and a point 212 at the working end of the instrument 200 which characterises the position of the working end of the instrument 200. As used herein, marked instrument refers to an instrument bearing a marker or markers by which the position of the instrument can be tracked by a tracking system.

The marker calibration vector 210 can be represented in practice by data specifying two vectors. The first vector extending perpendicularly from the centre of the plane defined by the arms of the marker array 208 towards the longitudinal axis of the instrument. The second vector comprises the remaining component of the calibration vector 210, that is a vector extending along the longitudinal axis of the instrument to the working point 212.

Figure 3 shows an instrument 220 according to the present invention which is similar to instrument 200 shown in Figure 2 but includes a single calibration point by which the initial calibration of the instrument can be checked. The initial calibration of the instrument is either the positional relationship according to manufacturing specifications or the positional relationship according to a prior calibration of the instrument. A formation in the form of a dimple 222 is located at the working point of the instrument and which in this instance is on the longitudinal axis of the instrument. The dimple is sized to have substantially the same size as the tip of a trackable pointer which is used to identify the position of the working point to a tracking system. This ensures that the position of the calibration reference point can be accurately determined.

As will be appreciated in this embodiment, the calibration formation 222 is coincidental with the working point 212 of the instrument and therefore has a fixed positional relationship therewith. Initially, the positional relationship between the centre of the marker array 208 and the calibration formation 222 is stored in database 118 of the computer system to define the marker calibration vector 226.

Figure 4 shows the instrument 220 of Figure 3, but after the instrument has undergone some wear or damage during use so that the configuration of the instrument no longer corresponds to the original configuration of the instrument (as shown in dashed lines). As the original calibration vector 226 stored by the CAS system no longer corresponds to the actual position of the working point of the instrument relative to the marker array 208, as represented by vector 228, navigated use of the instrument will result in inaccuracies. However, the instrument of the present invention, and associated method of the invention allows the calibration easily and quickly to be checked intra-operatively. Additionally, the invention allows the calibration of the instrument to be initially determined..

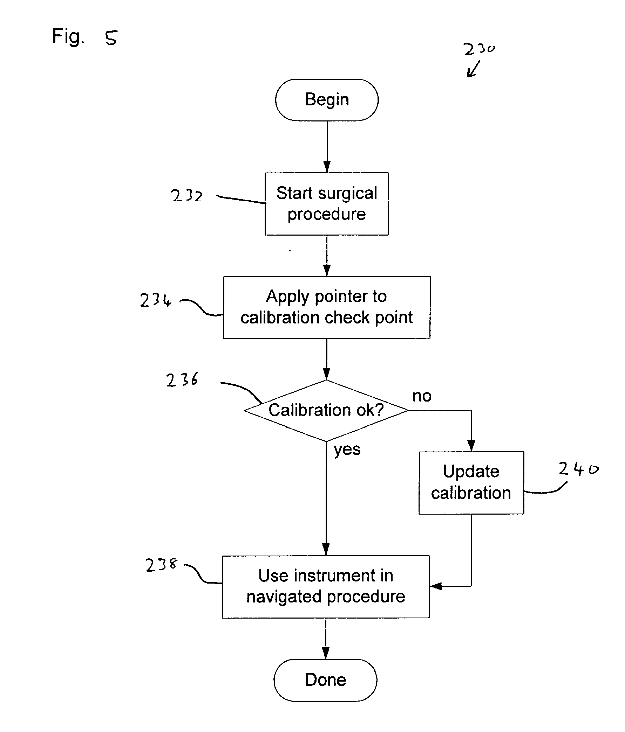

Figure 5 shows a flowchart illustrating a method 230 for checking the calibration of instrument 220 according to the invention which can be carried out by a surgeon or other member of the operating theatre team. The CAS procedure is begun at 232 and at some stage during the procedure, the surgeon or other member of the operating theatre team, can decide to check the calibration of an instrument to be used. At step 234, the individual uses a trackable pointer to allow the tracking system to determine the position of the calibration point 222. In particular, the individual places the tip of the trackable pointer into the dimple 222 at the working point of the instrument and the tracking system determines the actual position of the working point. The individual pivots the pointer about its tip in the dimple 222 and the tracking system captures a plurality of positions of the marker spheres to determine the centre of motion, which corresponds to the calibration reference point. The CAS system is tracking the position of the marker array also and so can determine what the current vector between the marker array and working point actually is. The CAS system 100 can display an indication of whether the calibration of the instrument is still accurate and at step 236 the individual can select whether to update the calibration of the instrument or whether the original calibration is still accurate.

If it is decided that the original calibration is still sufficiently accurate then at step 238 the instrument can be used in the navigational surgical procedure. If at step 236 it is decided that the original calibration is not accurate then at step 240 the CAS system can be instructed to use the newly defined calibration vector for the instrument so that use of the instrument at step 238 is navigated using the updated calibration vector rather than the original calibration vector.

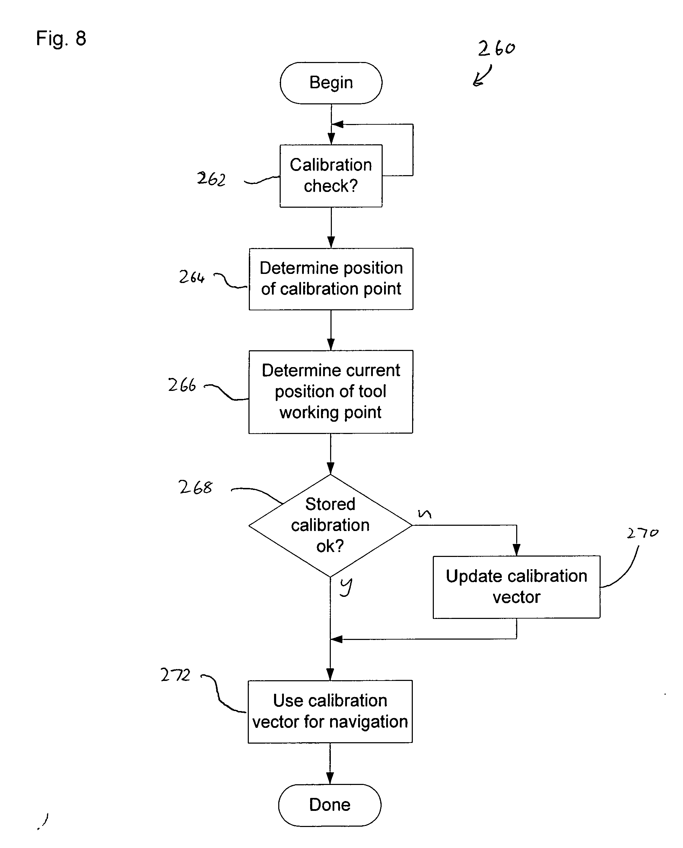

The data processing operations involved in enabling this procedure are described in greater detail below with reference to Figure 8.

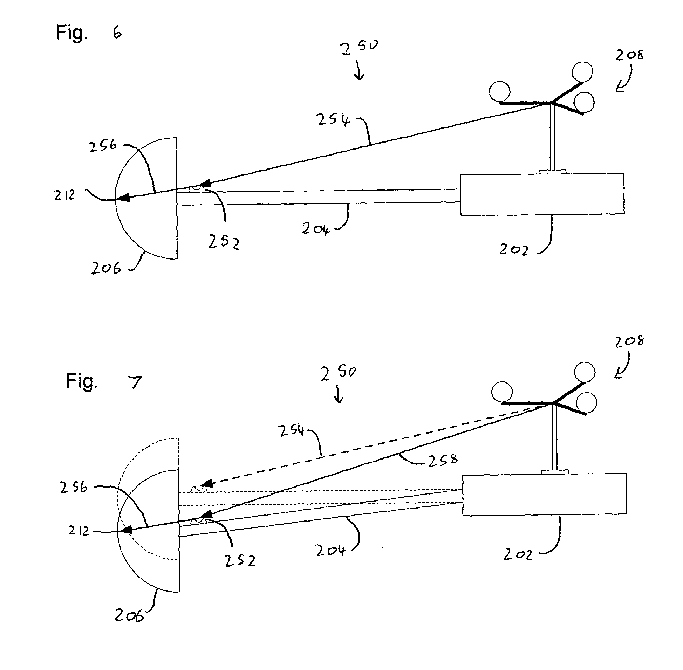

Figure 6 shows an alternate embodiment of a rotationally symmetric instrument 250 according to the invention. Instrument 250 is similar to instrument 220 except that the calibration point formation 252 is offset from the actual position of the working point 212 of the instrument. As illustrated in Figures 6 and 7, the formation 252 defining the calibration point is in the form of a dimple on the body 204 located adjacent or near the working end of the instrument.

When initially calibrated, data representing the positional relationship between the marker 208 and the calibration formation 252, i.e. representing vector 254, is generated and stored in the CAS system. Also, data representing the substantially fixed positional relationship between the calibration formation 252 and the working point 212, i.e. representing vector 256, is also generated and stored. Therefore when initially calibrated, the positional relationship between the working point 212 and the marker 208 is provided by the resultant of vectors 254 and 256.

Figure 7 shows the same instrument 250, but after use or damage such that the configuration of the instrument has changed. As the calibration formation 252 is located adjacent the working end of the instrument, it can be assumed that the positional relationship between the calibration formation 252 and the working point 212 is substantially constant, that is that vector 256 can be considered to be constant. However as illustrated, there is a significant difference in the positional relationship between the marker 208 and calibration formation 252, as illustrated by vectors 254 and 258.

Therefore, in order to check the calibration of the instrument intra-operatively, the surgeon merely needs to locate the tip of a marked pointer in the dimple of calibration formation 252 and the CAS system determines vector 258. The CAS system can then determine whether vector 258 sufficiently closely matches the original vector 254 in which case re-calibration of the instrument is not required. If not, then the instrument can be re-calibrated by using vector 258 to determine the overall calibration vector being the resultant of new vector 258 and original vector 256. Therefore, use of the instrument 250 shown in Figures 6 and 7 is similar to use of instrument 220 shown in Figures 3 and 4.

With reference to Figure 8 there is shown a process flowchart illustrating a data processing method 260 implemented in software for checking the calibration of an instrument. Process 260 can be provided as a routine or method which can be called or invoked by CAS application 110 when a user of the CAS system enters a command or instruction to check the calibration of an instrument, or alternatively if the workflow of the CAS application automatically requires a user to check the calibration of an instrument to be used. At step 262, the process waits until the calibration process 260 is called.

The tracking system can identify each different marked entity by virtue of the different geometries of the markers attached to them. The data allowing the CAS system to distinguish between the tracked entities is stored in database 118 for each entity being tracked. The tracking system continuously monitors the position of the marker 208 attached to the instrument and therefore has access to data indicating the position of the centre of the marker array. The tracking system also tracks the position of a pointer used by the surgeon, as described previously, to identify the position of the calibration formation 222, 252. At step 264, the user can enter a command instructing the CAS system to capture the current position of the pointer, while the tip of the pointer is engaged in the dimple of the calibration formation, from which the position of the calibration point can be determined.

Then at step 266, the current actual position of the tool working point is determined. For the instrument in Figures 3 and 4, this will simply correspond to the position of the tip of the pointer which is derived from the tracked pointer marker position. For an instrument 250 illustrated in Figures 6 and 7, the current position of the instrument working point is the resultant of the position of the tip of the pointer, again derived from the tracked position of the pointer's marker, and vector 256, which is obtained from database 118 as an attribute of the instrument. Then at step 268, it is determined whether the currently determined position of the working point of the instrument sufficiently closely matches the original position of the tool working point as stored in database 118. That is, it is determined whether the current calibration vector sufficiently closely matches the original calibration vector. A simple threshold mechanism method can be applied such that the calibration of the instrument is considered still to be acceptable if the newly determined calibration vector does not differ from the previously stored calibration vector by more than any inherent system noise or by more than a portion or percentage of the original vector.

If the calibration of the instrument is considered no longer to be sufficiently accurate then the system can store data representing the new calibration vector at step 270 in database 118. Alternatively, a message can be displayed to the user requiring user input to confirm whether to store the new calibration vector or whether to recheck the calibration of the instrument. If it is determined at step 268 that the calibration of the instrument sufficiently closely matches its original calibration, then processing can proceed directly to step 272. The original calibration vector, or newly updated calibration vector, is then used during subsequent navigation of the instrument to ensure that the navigation is accurate. The calibration checking routine then terminates.

As will be appreciated, this approach assumes that the array attachment point relative to the centre of the navigation array 208 moves much less due to manufacturing tolerances or usage than the working end of the instrument moves relative to the attachment point of the marker array 208. On this basis, the newly calibrated instrument working point can be used during navigation. The lesser movement of the array attachment point can be considered a reasonable assumption in practice since the largest manufacturing tolerances are usually with respect to the angular orientation of the navigation array rather than its exact spacial position. Angular tolerances have negligible effect on the position of the centre of the array. Further, the shortest distance of the array centre to the instrument axis will in general be much less than the distance to the instrument tip and therefore the variation in angle due to bending will be much less.

For the embodiment shown in Figures 6 and 7, providing the calibration formation close to the working point and axis of the instrument, compared to the distance to the navigation array, means that vector 256 is likely to be only negligibly affected by any bending of the instrument. Hence the instrument calibration can be checked and corrected by detecting the position of the calibration point and adding the working point offset vector 256 to the marker array position vector 258 in order to check the instrument calibration.

It will be appreciated that as only a single point needs to be captured in order to check the calibration of the instrument, the checking calibration of the instrument using the method of the present invention is very simple and quick.

It will be further understood, that this invention is not limited to the specific instrument shown in the Figures and can be used with any rotationally symmetric instrument. Owing to the rotational symmetry of the instrument it is necessary to capture the position of only one calibration point, from which the position of the working point 212 of the instrument can be determined. For example, the present invention could be used with the various rotationally symmetric instruments, such as, for example, inserters, pointers, a cup inserter, a cup reamer, drill guides (for screws, rods or anything else guided along an axis), instruments used in spinal applications or navigated arthroscopes.

## Claims

1. A rotationally symmetric surgical instrument for use with a computer aided surgery system, the instrument including a body having a working end in which a working point characterises the location in space of the working end, the instrument also having a location at which a marker can be attached to the instrument and only a single formation which provides a calibration reference point located toward the working end and which has a substantially fixed position relative to the working point for engaging with a trackable instrument so that the position of the calibration reference point can be determined.

2. An instrument as claimed in claim 1, wherein the formation comprises a dimple.

3. An instrument as claimed in claim 1, wherein the calibration reference point is coincidental with the working point.

4. An instrument as claimed in any preceding claim, wherein the calibration reference point lies on a longitudinal axis of the instrument.

5. An instrument as claimed in claim 1, 2 or 4, wherein the calibration reference point is off set from a longitudinal axis of the instrument.

6. An instrument as claimed in any preceding claim, wherein the calibration reference point is located within approximately the last third of the longitudinal length of the instrument at the working end of the instrument.

7. A computer implemented method for checking the calibration of a rotationally symmetric surgical instrument being used with a computer aided surgery system, the instrument having a working point which characterises the position of a working end of the instrument and only a single formation providing a calibration reference point, and wherein a trackable marker is attached to the instrument, the method comprising:
determining the position of the trackable marker attached to the instrument;
determining the position of the calibration reference point of the instrument from the tracked position of a further instrument while engaging the formation; and
using the position of the calibration reference point to determine whether the actual position of the working point relative to the position of the trackable marker corresponds to a calibration position of the working point relative to the position of the trackable marker.

8. The method as claimed in claim 7, wherein if the actual position does not correspond to the calibration position, then:
storing new calibration data representing the actual position of the working point relative to the position of the trackable marker; and
using the new calibration data to determine the position of the working point

9. The method as claimed in claim 7 or 8, further comprising reading stored data representing the position of the calibration reference point relative to the working point.

10. A computer aided surgery system which can check the calibration of a rotationally symmetric surgical instrument being used with the computer aided surgery system, the instrument having a working point which characterises the position of a working end of the instrument and only a single formation providing a calibration reference point, and wherein a trackable marker is attached to the instrument, the computer aided surgery system including a data processing device and a medium storing computer program code providing instructions which can cause the system to:
determine the position of the trackable marker attached to the instrument;
determine the position of the calibration reference point of the instrument from the tracked position of a further instrument while engaging the formation; and
use the position of the calibration reference point to determine whether the actual position of the working point relative to the position of the trackable marker corresponds to a calibration position of the working point relative to the position of the trackable marker.

11. A method for checking the calibration of a rotationally symmetric instrument during a computer aided surgical procedure, the instrument having a working point which characterises the position of a working end of the instrument and only a single formation providing a calibration reference point, and wherein a trackable marker is attached to the instrument, the method comprising:
engaging a further trackable instrument with the single formation only; and
causing the position of the calibration reference point to be determined.

12. The method as claimed in claim 11, and further comprising instructing a computer aided surgery system whether to re-calibrate the surgical instrument.

13. Computer program code executable by a data processing device to carry out the method of any of claims 7 to 9 or provide a computer aided surgery system as claimed in claim 10.

14. A computer readable medium bearing computer program code as claimed in claim 13.
